# DEMANDE DE BREVET EUROPEEN

(11) **EP 4 303 320 A1**
(43) Date de publication de la demande: **10.01.2024**
(21) Numéro de dépôt: 22183395.7
(22) Date de dépôt: 06.07.2022
(51) Int. Cl.: C12Q 1/6883

(54) **DÉTERMINATION DU RISQUE DE DÉCÈS D'UN SUJET INFECTÉ PAR UN VIRUS RESPIRATOIRE PAR MESURE DU NIVEAU D EXPRESSION DU GÈNE TDRD9**

(71) Demandeur: BIOMÉRIEUX, 69280 Marcy L'Etoile (FR); Hospices Civils de Lyon, 69002 Lyon (FR); UNIVERSITE CLAUDE BERNARD - LYON 1, 69100 Villeurbanne (FR)
(72) Inventeur: TARDIVEAU, Claire, 69650 Saint Germain au Mont d'Or (FR); LUKASZEWICZ, Anne-Claire, 69006 LYON (FR); VENET, Fabienne, 69003 LYON (FR); MONNERET, Guillaume, 69003 LYON (FR)
(74) Mandataire: bioMérieux PI Groupement mandataires

(57) **Abrégé**

L'invention concerne un procédé de détermination *in vitro* ou ex *vivo* du risque de décès chez un sujet infecté par un virus respiratoire, par exemple le SARS-CoV-2, comprenant une de mesure, dans un échantillon biologique dudit sujet, du niveau d'expression du gène TDRD9, ainsi que les kits associés.

## Description

### DOMAINE TECHNIQUE

La présente invention concerne le domaine technique des procédés *in vitro* et kits de diagnostic. En particulier, l'invention a pour objet des procédés et kits permettant de déterminer le risque de décès d'un sujet infecté par un virus respiratoire, notamment par un virus respiratoire tel que le SARS-CoV-2 ou un de ses variants.

### TECHNIQUE ANTERIEURE

La pandémie de la maladie liée au coronavirus (COVID-19) causée par le coronavirus 2 du syndrome respiratoire aigu sévère (SARS-CoV-2) a infecté à ce jour plus de 550 millions de patients dans le monde et provoqué plus de 6,3 millions de décès.

La gravité de la maladie varie considérablement d'un patient à l'autre et la majorité des patients est asymptomatique ou présente des symptômes minimes tels que la fièvre, la toux et/ou un essoufflement. Néanmoins, 5 à 10 % des patients nécessitent des soins intensifs en raison de l'évolution rapide (9 à 12 jours) vers une forme grave ou critique avec le développement, par exemple, de syndrome de détresse respiratoire aiguë (SDRA) et/ou hypoxémie sévère, de lésions pulmonaires aiguës (LPA), de défaillance de plusieurs organes, voire de décès (C. Huang *et al.,* 2020).

La réponse immunitaire joue un rôle prépondérant dans la physiopathologie de la COVID-19 et le phénotype le plus sévère lors de l'admission en soins intensifs des patients infectés se caractérise par un profil immunitaire complexe qui évolue au fil du temps (E.Z. Ong *et al.,* 2020).

Malgré tout, chez les patients sévères atteints du COVID-19, la réponse immunitaire peut être définie par une altération des réponses inflammatoires et immunitaires avec une lymphopénie marquée, un nombre élevé de neutrophiles et de monocytes, une diminution de l'expression HLA-DR des monocytes, une tempête modérée de cytokines plasmatiques, une réponse inadéquate de la signalisation des interférons de type I et une régulation négative des gènes stimulés par I'IFN(ISG) (F. Venet *et al.,* 2021).

Au fil des mois, plusieurs groupes de recherches ont essayé d'identifier les facteurs de risques caractéristiques d'une évolution sévère de la maladie COVID-19 afin notamment d'identifier les patients à haut risques de développer lesdites formes sévères.

Par exemple, dans une étude combinant une cinquantaine de caractéristiques cliniques et environ deux cents caractéristiques immunologiques de haute dimensionnalité, trois immunotypes distincts associés à la gravité de la maladie ont été décrits (Mathew et al., 2020).

Dans le cadre d'une vaste étude d'évaluation immunitaire combinant des données cellulaires obtenues par cytométrie en flux, des marqueurs immunitaires solubles (analyse multiplex des cytokines), l'expression de I'ARN (Nanostring) et la sérologie (ELISA), une signature immunitaire centrale du sang périphérique chez les patients atteints de COVID-19 a été découverte, ladite signature pouvant permettre d'identifier les paramètres de l'immunopathologie, de corréler avec la gravité de la maladie mais aussi d'anticiper la progression clinique (Laing *et al.,* 2020).

Plus récemment, il a également été établi que les trajectoires longitudinales de 11 biomarqueurs circulants basés sur l'immunité pouvaient être associées à la mortalité des patients lorsqu'elles étaient augmentées (10) ou diminuées (1), fournissant ainsi un début de preuve que des biomarqueurs basés sur l'immunité pourraient permettre d'obtenir une alerte précoce de l'issue des patients atteints par la COVID-19 (Abers *et al.,* 2021). Des profils d'expression géniques ont également été décrits pour prédire l'issue des patients atteints de la COVID-19 (Guardela B *et al.,* 2021).

Dans ce contexte, le biomarqueur CD177 a été décrit comme pouvant être associé à la sévérité et au risque de décès des patients atteints du COVID-19. Particulièrement, la stabilité des niveaux protéiques de CD177 dans le sérum chez les patients atteints de COVID-19 sévère au cours de la maladie est décrit comme le signe d'un pronostic plus défavorable, pouvant conduire au décès (Lévy Y *et al.,* 2021).

Néanmoins, et au regard de la prévalence mondiale, il subsiste toujours un besoin d'identifier de nouveaux biomarqueurs offrant d'autres alternatives pour prédire efficacement le risque de mortalité des sujets infectés par des virus respiratoires, notamment de virus respiratoires responsables de la COVID-19, afin de pouvoir adapter la prise en charge, préférentiellement de manière précoce, au travers de thérapies guidées et d'améliorer ainsi leurs chances de survie.

### RESUME DE L'INVENTION

Un premier objet de l'invention concerne un procédé in vitro ou ex vivo pour déterminer le risque de décès chez un sujet infecté par un virus respiratoire, comprenant une étape de mesure, dans un échantillon biologique dudit sujet, du niveau d'expression du gène TDRD9, et éventuellement de l'expression d'au moins un autre gène additionnel choisi parmi CD74, IL1R2 et/ou CD177.

Avantageusement, le procédé selon l'invention peut également en outre comprendre la mesure de l'expression d'un ou plusieurs gènes additionnels impliqués dans la réponse de l'hôte tels que ceux listés dans le tableau 2.

Le procédé selon l'invention permet ainsi de manière tout à fait avantageuse de déterminer le risque de décès d'un sujet infecté par un virus respiratoire, notamment un virus respiratoire tel que le SARS-CoV-2 ou un de ses variants.

La conclusion du risque de décès est notamment établie en comparant le niveau d'expression de TDRD9 à celui d'une valeur de référence qui peut correspondre à la moyenne du niveau d'expression de TDRD9 obtenu à partir d'échantillons biologiques issus d'une population de sujets ne présentant aucune infection par un virus respiratoire ou à la moyenne du niveau d'expression de TDRD9 obtenue à partir d'échantillons biologiques issus d'une population de sujets infectés par un virus respiratoire et dont on sait qu'ils ont survécus après l'infection, notamment dans les 28 jours suivants l'infection.

En particulier, sur la base du résultat de la comparaison du niveau d'expression de TDRD9 avec une valeur de référence, une conclusion quant à la présence d'un risque accru de décès chez le sujet est émise dès lors qu'une augmentation de l'expression est identifiée.

Un autre objet de l'invention concerne un kit pour la mesure in vitro ou ex vivo, de l'expression de TDRD9 dans un échantillon biologique comprenant des moyens de détermination du niveau d'expression de TDRD9 dans ledit échantillon. Le kit peut également comprendre des moyens de détermination du niveau d'expression d'au moins un autre gène additionnel sélectionné parmi CD74, IL1R2 et CD177, et des moyens de déterminations d'au moins un gène impliqué dans la réponse de l'hôte tels que ceux listés dans le tableau 2.

De manière avantageuse, le kit peut notamment comprendre un échantillon contrôle positif calibré pour contenir la quantité de TDRD9 qui correspond à la quantité ou la concentration représentative du niveau d'expression mesuré dans un pool d'échantillons de sujets ne présentant pas d'infection par un virus respiratoire, et/ou un échantillon contrôle négatif calibré pour contenir la quantité de TDRD9 qui correspond à la quantité moyenne mesurée dans un pool d'échantillons de sujets qui n'ont pas survécu suite à une infection par un virus respiratoire.

Un autre objet de l'invention concerne l'utilisation du kit pour la détermination du risque de décès, de préférence du risque de décès dans les 28 jours post-infection, d'un sujet infecté par un virus respiratoire tel que le SARS-CoV-2 ou un de ses variants.

### DESCRIPTION DES FIGURES

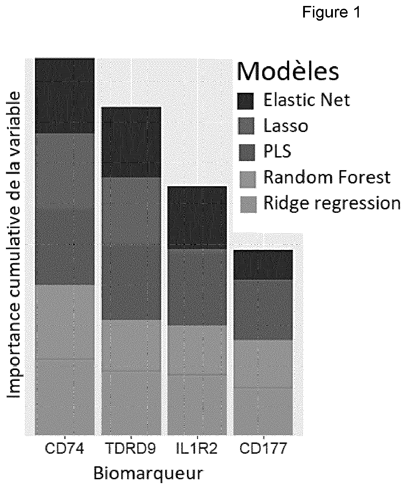

La figure 1 est un diagramme représentant l'importance des variables dans différents modèles d'apprentissage machine (« Machine Learning ») utilisés pour prédire la mortalité dans les 28 jours au sein de la sous-cohorte RICO. Les marqueurs TDRD9, CD74, et ILR2 ont été identifiés dans les 5 modèles et TDRD9 présente de bons résultats. CD177 n'a pas été capturé par le modèle Lasso mais a été le quatrième marqueur d'intérêt identifié par l'importance cumulative de la variable.

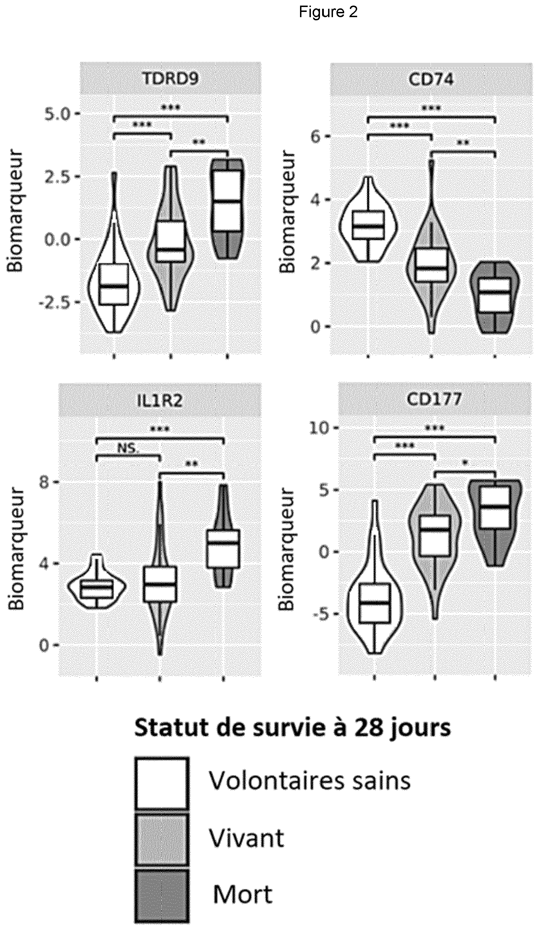

La figure 2 concerne les diagrammes en violon représentant l'expression de TDRD9 et de gènes additionnels associés à la mortalité et identifiés par les modèles d'apprentissage automatiques. Les valeurs p ont été calculées avec un test de Wilcoxon. A gauche, les volontaires sains, au milieu les patients vivants au 28e jour après l'admission et à droite, ceux qui sont décédés avant le 28e jour.

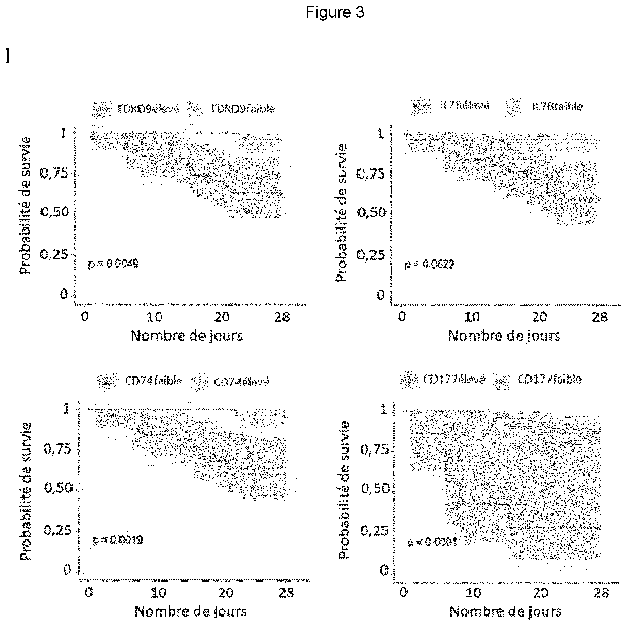

La Figure 3 décrit les associations entre les niveaux d'ARNm de TDRD9, CD177, CD74, et IL1R2 mesurés à l'admission et la survenue de décès dans les 28 jours suivant l'admission. Les événements cumulatifs non ajustés pour le décès ont été obtenus avec des estimations de Kaplan-Meier. Les patients ont été stratifiés en 2 groupes sur la base du seuil de Youden déterminé pour chaque ARNm pour la prédiction de la mortalité dans à 28 jours dans la population totale des patients à l'admission. La valeur p du test de Log-Rank est indiquée.

### DESCRIPTION DETAILLEE DE L'INVENTION

Certains termes et expressions utilisés dans le cadre de l'invention sont détaillés ci-après.

Un premier objet de l'invention concerne un procédé *in vitro* ou ex *vivo* pour déterminer le risque de décès chez un sujet infecté par un virus respiratoire, comprenant une étape de mesure, dans un échantillon biologique dudit sujet, du niveau d'expression du gène TDRD9.

D'une manière tout à fait surprenante, il a été constaté que la mesure de l'expression du gène TDRD9 permettait de déterminer le risque de décès chez un sujet infecté par un virus respiratoire. Ainsi, et dans un contexte où la médecine personnalisée prend de plus en plus d'importance, les sujets présentant un risque accru de mortalité pourraient bénéficier d'une prise en charge individualisée.

Cela est d'autant plus surprenant que chez un même sujet infecté, les paramètres immunologiques standards mesurés à l'admission en soins intensifs et utilisés comme références par les praticiens, à savoir I'HLA-DR monocytaire et les interleukines plasmatiques IL-6 et IL-10, ne sont pas significativement associés à la mortalité au 28 jours après l'admission. Cela démontre ainsi tout l'intérêt de mesurer le niveau d'expression du gène TDRD9 et des gènes additionnels selon l'invention.

Le procédé objet de l'invention présente l'avantage de pouvoir facilement évaluer le risque de décès d'un sujet, par exemple un patient admis en service de réanimation ou aux urgences, en disposant d'un biomarqueur directement mesurable et dont la mesure peut être faite directement dans l'établissement de santé l'accueillant ou dans un laboratoire de proximité. De plus, la mesure du biomarqueur TDRD9, ou des autres biomarqueurs additionnels de l'invention, est tout à fait adaptée pour être réalisée par des automates d'analyse ou par des tests dits rapides.

Par biomarqueur (ou « marqueur »), on entend une caractéristique biologique mesurable objectivement qui représente un indicateur des processus biologiques normaux ou pathologiques ou de réponse pharmacologique à une intervention thérapeutique. Au sens de la présente description, les biomarqueurs sont des gènes et leur niveau d'expression est détectable en particulier au niveau des transcrits, notamment des transcrits ARNm.

Le gène TDRD9 se trouve sur le chromosome 14 et code pour la protéine 9 contenant le domaine Tudor (TDRD9) qui est une ARN hélicase normalement exprimée dans la lignée germinale. Cette hélicase agit par l'intermédiaire du processus métabolique des piARN qui assure la répression des éléments transposables au cours de la méiose par l'intermédiaire de la formation de complexes composés de piARN et de protéines Piwi.

La séquence du gène TDRD9 est accessible auprès du NCBI sous la référence NC_000014.9 .

L'expression « risque de décès chez un sujet » fait référence au risque que le sujet a de succomber dans les jours suivants l'infection par un virus respiratoire ou dans les jours suivants l'admission en établissement de santé consécutive à l'infection. On parle notamment de risque de décès accru lorsque le sujet présente un risque statistiquement significatif de décès dans les jours qui suivent l'infection, généralement par rapport à des sujets infectés dont on sait qu'ils ont survécus ou à des sujets non infectés.

De manière avantageuse, le procédé selon l'invention permet de déterminer le risque de décès chez un sujet dans les 28 jours suivants le jour où l'infection est avérée, également appelés jours post-infection, notamment par un test de détection d'un virus respiratoire.

Le terme « sujet » désigne un être humain et de préférence, le sujet est un patient. Le patient est une personne entrée en contact avec un professionnel de santé, notamment un médecin, une structure médicale ou un établissement de santé.

Selon un mode de réalisation particulier, le sujet est un patient au sein d'un établissement de santé, de préférence au sein d'un hôpital, de préférence encore au sein du service des urgences, du service de réanimation, en unité de soins intensifs (USI) ou en unité de soins continus, et tout particulièrement un patient en USI.

Par « virus respiratoire », on entend un virus qui infecte les voies respiratoires et/ou les poumons. De tels virus se retrouvent classiquement dans les échantillons prélevés dans le nez, la gorge et/ou la bouche d'un sujet, notamment dans les échantillons nasaux ou naso-pharyngés (qui nécessitent un prélèvement plus profond dans le nez), les échantillons oro-pharyngés (qui nécessitent un prélèvement au fond de la gorge) ou la salive.

Par l'expression « sujet infecté par un virus respiratoire », on entend un sujet dont le test de détection de la présence d'un virus respiratoire donne un résultat positif. Ces sujets, notamment ceux développant les formes les plus graves de la maladies, sont des sujets pour lesquels il est d'autant plus pertinent de mettre en oeuvre le procédé selon l'invention mesurant le niveau d'expression de TDRD9.

A l'inverse, un sujet non-infecté par un virus respiratoire est un sujet dont le test de détection de la présence d'un virus respiratoire est négatif.

A titre d'exemple de virus respiratoire, on peut citer les coronavirus saisonniers, le virus SARS-CoV-2 (« Severe Acute Respiratory Syndrome Coronavirus-2 »), quels que soient ses variants, le virus de la grippe, le virus respiratoire syncytial (VRS), les rhinovirus, les métapneumovirus, les virus parainfluenza et les adénovirus. A ce jour, les variants connus du virus SARS-CoV-2, sont, notamment ses variants dits anglais, brésilien, sud-africain, américain, indien ou Omicron. La connaissance de ces variants et leurs dénomination évoluent et l'invention est applicable à chacun d'entre eux (https://www.who.int/en/activities/tracking-SARS-CoV-2-variants/).

La découverte du « variant anglais » de SARS-CoV-2 remonte au 20 septembre 2020, dans le Kent (Sud-Est de l'Angleterre). Le 14 décembre, le Royaume-Uni a signalé à l'Organisation mondiale de la santé (OMS) la circulation de ce variant. Baptisé dans un premier temps VUI 202012/01 (pour *Variant Under Investigation,* année 2020, mois 12, variant 01), il est rapidement renommé, le 18 décembre 2020, VOC 202012/01 (pour *Variant Of Concern,* littéralement « variant préoccupant »), puis variant Alpha. Il fait partie du lignage B.1.1.7 dans l'arbre phylogénétique et comporte la délétion 69-70, encore désignée ΔH69/V70. Le variant brésilien P.1 (variant Gamma) est un descendant du lignage B.1.1.28. Ce variant brésilien P.1 renferme de nombreuses mutations, en particulier les mutations E484K, K417T et N501Y. Le variant japonais qui dérive d'un lignage présent au Brésil (B.1.1.28) renferme un nombre très élevé de changements génétiques. Il comporte douze mutations d'acides aminés dans la protéine *spike,* notamment les mutations N501Y, E484K, K417T. Le variant sud-africain (variant Beta) baptisé 501Y.V2 et appartenant au lignage B.1.351 renferme lui aussi différentes mutations dont trois, K417N, E484K et N501Y, localisées dans le domaine RBD de la protéine *spike,* le domaine de fixation au récepteur. Le variant Indien, baptisé variant Delta qui appartient au lignage B. 1.617.2 et renferme les mutations S417N et S484K. Enfin, il y a également le variant Omicron identifié en novembre 2021 et qui appartient au lignage B.1.1.529.

Selon un mode de réalisation particulier, le virus respiratoire est un coronavirus, en particulier le SARS-CoV-2 ou un de ses variants tels que par exemple les variants Alpha, Beta, Gamma, Delta ou Omicron.

Les expressions « test de détection d'un virus respiratoire », « test de diagnostic d'un virus respiratoire » ou « test de détection de la présence d'une infection par un virus respiratoire », sont synonymes et font référence à tout test connu de l'homme du métier permettant d'apporter une telle conclusion, notamment les tests de détection de l'ADN ou de l'ARN dudit virus respiratoire, tels que les tests PCR, les tests antigéniques ou encore les auto-tests.

Par « échantillon biologique », on se réfère ici à tout échantillon provenant d'un sujet, et pouvant être de différentes natures, comme le sang ou ses dérivés, les expectorations, l'urine, les selles, la peau, le liquide céphalo-rachidien, le liquide de lavage broncho-alvéolaire, le liquide de ponction de la cavité abdominale, la salive, les sécrétions gastriques, le sperme, le liquide séminal, les larmes, la moelle épinière, ganglion du nerf trijumeau, tissu adipeux, tissu lymphoïde, tissu placentaire, tissu du tractus gastro-intestinal, tissu du tractus génital, ou le tissu du système nerveux central.

En particulier, l'échantillon biologique peut être un fluide biologique, comme un échantillon de sang ou un échantillon dérivé du sang, qui peut notamment être choisi parmi le sang total (tel que collecté par voie veineuse, c'est-à-dire contenant les cellules blanches et rouges, les plaquettes et le plasma), le plasma, le sérum, ainsi que tous les types de cellules extraites à partir du sang, comme par exemple les cellules mononuclées sanguines périphériques (ou PBMC, contenant les lymphocytes B, les lymphocytes T, les cellules NK, les cellules dendritiques et les monocytes), des sous-populations de cellules B, des monocytes purifiés, ou encore des neutrophiles.

Selon un mode de réalisation préféré, l'échantillon biologique mis en oeuvre dans le procédé selon l'invention est un échantillon de sang, de préférence un échantillon de sang total.

Au sens de la présente description, l'échantillon biologique d'un sujet, à savoir celui d'un sujet infecté dont on souhaite déterminer le risque de décès, correspond à l'échantillon biologique à tester, ou échantillon test, par opposition à l'échantillon de référence utilisé comme comparatif.

Au sens de la présente description, l'expression « valeur de référence » ou « valeur de référence prédéterminée », est synonyme des expressions « valeur contrôle » ou « valeur seuil », et sert de point de comparaison pour déterminer si le niveau d'expression d'un gène cible est diminué ou augmenté.

Selon le procédé objet de l'invention, le risque de décès chez un sujet infecté par un virus respiratoire est déterminé au travers de la mise en oeuvre d'une étape de mesure du niveau d'expression du gène TDRD9.

La mesure du niveau d'expression d'un gène est bien connue de l'homme du métier et consiste notamment à quantifier au moins un produit d'expression du gène. Le produit d'expression d'un gène, au sens de la présente invention, peut être toute molécule biologique issue de l'expression dudit gène. Plus particulièrement, le produit d'expression du gène peut être un transcrit.

Par « transcrit », on entend les ARN, et en particulier les ARN messagers (ARNm), issus de la transcription du gène. Plus précisément, les transcrits sont les ARN produits par la transcription d'un gène suivi des modifications post-transcriptionnelles des formes pré-ARN.

Selon un mode de réalisation préféré, la mesure du niveau d'expression de TDRD9, et éventuellement celle d'un ou plusieurs autres gènes additionnels tels que définis ci-après, est réalisée au niveau ARN, en particulier au niveau messager (ARNm), dans un échantillon biologique d'un sujet infecté par un virus respiratoire. Selon ce mode de réalisation, la mesure du niveau d'expression du gène TDRD9 concerne donc la détermination du niveau d'ARNm dudit gène.

Comme mentionné précédemment, la mesure du niveau d'expression d'un gène est bien connue de l'homme du métier. Dans le cas d'un transcrit, notamment I'ARNm, la mesure peut être réalisée par une méthode directe, selon tout procédé connu de l'homme du métier permettant de déterminer la présence dudit transcrit dans l'échantillon biologique, ou par détection indirecte du transcrit après transformation de ce dernier en ADN, ou après amplification dudit transcrit ou après amplification de I'ADN obtenu après transformation dudit transcrit en ADN. De nombreuses méthodes existent pour la détection des acides nucléiques et sont bien connues de l'homme du métier (voir par exemple Kricka et al., Clinical Chemistry, 1999, n° 45(4), p.453-458 ; Relier G. H. et al., DNA Probes, 2nd Ed., Stockton Press, 1993, sections 5 et 6, p.173-249).

L'expression des gènes peut notamment être mesurée par Reverse Transcription-Polymerase Chain Reaction ou RT-PCR, de préférence par RT-PCR quantitative ou RT-qPCR (par exemple en utilisant la technologie FilmArray^{®} ou la plateforme BiomarkTM de Fluidigm), par séquençage (de préférence par séquençage haut débit) ou par des techniques d'hybridation (par exemple avec des micropuces d'hybridation ou par des techniques du type NanoString^{®} nCounter^{®}). Toutes ces méthodes sont également bien connues de l'homme du métier et il n'est pas nécessaire de les détailler ici.

Selon un mode de réalisation particulier, la mesure de l'expression de TDRD9 est réalisée par une méthode de détection moléculaire, notamment par RT-PCR, par séquençage, ou par hybridation. De préférence, la mesure de l'expression est réalisée par RT-PCR, et notamment par RT-qPCR.

Selon un mode de réalisation particulier, le niveau d'expression de TDRD9 est mesuré par détection quantitative RT-qPCR des transcrits ARNm de TDRD9.

La mesure du niveau d'expression permet de déterminer la quantité d'un ou plusieurs transcrits de TDRD9 dans l'échantillon biologique ou également d'en donner une valeur dérivée.

Ainsi, selon un mode de réalisation particulier, le niveau d'expression du gène TDRD9 est une valeur dérivée de la quantité de ses transcrits. A titre d'exemple, une valeur dérivée de la quantité des transcrits peut par exemple être la concentration absolue, calculée grâce à une courbe de calibration obtenue à partir de dilutions successives d'une solution d'amplicons de concentration connue. Elle peut également correspondre à la valeur de la quantité normalisée et calibrée, comme le CNRQ (Calibrated Normalized Relative Quantity, (Hellemans et al (2007), Genome biology 8(2):R19), qui intègre les valeurs d'un échantillon de référence (ou d'un calibrateur) et d'un ou plusieurs gènes de ménage (appelés également gènes de référence). A titre d'exemples de gènes de ménage, on peut citer les gènes DECR1, HPRT1, PPIB, RPLP0, PPIA, GLYR1, RANBP3, 18S, B2M, TBP, GAPDH et ACTB.

D'une manière générale, dans le procédé selon l'invention, quels que soient ses modes de réalisation, le niveau d'expression de TDRD9, de préférence l'expression normalisée, dans l'échantillon biologique du sujet est comparé à une valeur de référence ou à l'expression du même gène, de préférence l'expression normalisée, obtenue dans un échantillon biologique de référence.

Selon un mode de réalisation particulier, l'expression de TDRD9 peut en effet être normalisée par rapport à l'expression d'un ou plusieurs gènes de ménage (ou gènes de référence) selon les méthodes connues de l'homme du métier. Ainsi, l'expression est normalisée en utilisant un ou plusieurs des gènes de ménage suivants : DECR1 (localisation chromosomique: chr8, 90001352-90053633), HPRT1 (localisation chromosomique: chrX, 134452842-134520513) et PPIB (localisation chromosomique: chr15:64155812-64163205), RPLP0 (localisation chromosomique: chr12 , 120196699-120201111), PPIA (localisation chromosomique: chr7, 44795960-44803117), GLYR1 (localisation chromosomique : chr16, 4803203-4847288), RANBP3 (localisation chromosomique : chr19 , 5916139-5978140), B2M (localisation chromosomique : chr15, 44711492-44718145), TBP (localisation chromosomique : chr6, 170554369-170572859), GAPDH (localisation chromosomique : chr12, 6534517-6538371) et ACTB (localisation chromosomique : chr14 , 5527148-5530601). Les localisations chromosomiques sont données selon le GRCh38/hg38. De préférence, l'expression est normalisée en utilisant un ou plusieurs des gènes de ménage choisis parmi : DECR1, HPRT1, PPIB, GAPDH et ACTB, et de préférence encore, choisis parmi DECR1, HPRT1 et PPIB.

Dans un tel cas, le niveau de référence utilisé est également normalisé au préalable, de la même manière. La normalisation, que ce soit pour le niveau de référence ou pour le niveau de transcrits de l'échantillon biologique à tester, est réalisée avant la comparaison, notamment avant le calcul d'un rapport entre le niveau de transcrits dudit échantillon à tester et le niveau de référence. Lorsqu'une valeur seuil différente d'un niveau de référence est utilisée pour émettre une conclusion, il pourra être tenu compte de cette normalisation, pour le choix de la valeur seuil.

Dans le cas où le niveau de transcrits de TDRD9 est normalisé par rapport au niveau de transcrits d'un ou plusieurs gènes de ménage, bien entendu, cela implique que le procédé selon l'invention inclut la détermination du niveau de transcrits du ou des gènes de ménage utilisé(s) pour la normalisation.

Selon un mode de réalisation particulier, le niveau d'expression du gène TDRD9 est déterminé à partir d'un échantillon biologique d'un sujet et il peut être conclu à un risque de décès accru chez ledit sujet lorsque la comparaison du niveau de transcrits, notamment ARNm, dudit gène TDRD9 avec une valeur seuil de référence prédéterminée, montre qu'il y a une différence avec ladite valeur seuil de référence correspondant audit gène TDRD9. En particulier, ladite différence correspond à un niveau de transcrits pour l'échantillon biologique à tester qui est supérieur à celui correspondant à la valeur seuil.

De manière avantageuse, selon une première variante, ladite valeur seuil correspond à un niveau d'expression de référence dudit gène TDRD9 qui est le niveau de transcrits dudit gène obtenu à partir d'un échantillon biologique d'un sujet ne présentant aucune infection par un virus respiratoire. Selon cette variante, la valeur seuil peut également correspondre à la moyenne du niveau d'expression de transcrits de TDRD9 obtenue à partir d'échantillons biologiques issus d'une population de sujets ne présentant aucune infection par un virus respiratoire. Selon une seconde variante, la valeur seuil correspond à un niveau d'expression de référence du gène TDRD9 qui est le niveau de transcrits dudit gène obtenu à partir d'un échantillon biologique d'un sujet infecté par un virus respiratoire mais dont on sait qu'il a survécu après l'infection, notamment dans les 28 jours suivants l'infection. Selon cette variante, la valeur seuil peut également correspondre à la moyenne du niveau d'expression de transcrits de TDRD9 obtenue à partir d'échantillons biologiques issus d'une population de sujets infectés par un virus respiratoire mais dont on sait qu'ils ont survécus après l'infection, notamment dans les 28 jours suivants l'infection.

De manière préférée, ladite différence entre le niveau de transcrits du gène TDRD9 déterminé dans l'échantillon test et le niveau de référence dudit gène (valeur seuil), correspond au fait que le niveau de transcrits dudit gène déterminé dans l'échantillon test est augmenté de manière significative, en particulier d'au moins 10%, d'au moins 20%, d'au moins 30 %, d'au moins 40%, d'au moins 50%, d'au moins 60%, d'au moins 70%, d'au moins 80% ou d'au moins 90% par rapport au niveau de référence, ou valeur seuil de référence, dudit gène TDRD9.

L'augmentation considérée comme pertinente pour apporter une conclusion sera fonction de la valeur seuil considérée, et notamment du niveau de référence utilisé comme valeur seuil de référence, et peut être adaptée par l'homme du métier. En particulier, si la valeur seuil de référence correspond au niveau de référence dudit gène TDRD9 qui est le niveau de transcrits dudit gène chez un sujet ne présentant aucune infection par un virus respiratoire, chez une population de sujets ne présentant aucune infection par un respiratoire, ou chez un sujet ou une population de sujets présentant une infection mais ayant survécus dans les 28 jours suivants l'infection, il pourra suffire que le niveau de transcrits TDRD9 déterminé dans l'échantillon test soit simplement supérieur à ladite valeur seuil de référence.

Selon l'invention, le procédé permet de conclure à un risque accru de décès du sujet lorsqu'une surexpression de TDRD9 est mise en évidence dans l'échantillon biologique à tester.

Par « surexpression », on entend une augmentation significative du niveau d'expression par rapport à une valeur seuil de référence. L'homme du métier est en mesure de déterminer le test statistique à utiliser pour déterminer cette valeur seuil de référence avec laquelle le niveau d'expression de TDRD9 doit être comparé. Les exemples de réalisation présentent une des méthodes possibles.

Selon ce mode de réalisation particulier, le procédé peut ainsi comprendre les étapes suivantes de :
- déterminer le niveau d'expression du gène TDRD9 par mesure de la quantité d'au moins un transcrit de TDRD9 dans l'échantillon biologique du sujet,
- comparer la quantité dudit transcrit déterminée pour ledit échantillon biologique ou une valeur dérivée de cette quantité, à une valeur de référence prédéterminée à partir d'un échantillon de référence, et
- établir une conclusion quant au risque accru de décès, à partir du résultat de la comparaison.

Selon ce mode de réalisation, l'échantillon de référence peut être par exemple un échantillon provenant d'un sujet ou un mélange d'échantillons de plusieurs sujets, lesdits sujets étant non-infectés par un virus respiratoire.

La valeur de référence peut notamment correspondre à une valeur moyenne du niveau d'expression de TDRD9 mesurée à partir de plusieurs d'échantillons issus chacun de différents sujets non-infectés par un virus respiratoire.

Selon une variante préférée de ce mode de réalisation, l'échantillon de référence est de même nature que l'échantillon biologique à tester ou tout du moins d'une nature compatible pour constituer une référence quant à la détermination du niveau d'expression de TDRD9.

Une « comparaison » ou une vérification d'une « différence » entre deux valeurs, ou niveaux de valeurs, peut être effectuée par toute technique connue. La comparaison ou la vérification d'une « différence » peut impliquer le calcul d'un rapport ou d'une différence.

Dans le cadre de l'invention, l'émission d'une conclusion quant au risque de décès chez le sujet dont est issu l'échantillon test, peut également être effectuée par toute technique automatisée, réalisée par un ordinateur ou assistée par ordinateur.

Le procédé tel que décrit précédemment, dans tous ses modes de réalisation, comprend, outre l'étape de mesure de l'expression du gène TDRD9, une étape de mesure, dans l'échantillon biologique du sujet, de l'expression d'au moins un, deux, ou trois gène(s) additionnel(s) sélectionné(s) parmi les gènes suivants : CD74, IL1R2 et CD177.

Les valeurs de référence ou valeur seuil du niveau d'expression des gènes additionnels peuvent être déterminées de la même manière que le gène TDRD9 et tel que précédemment décrit.

Les localisations chromosomiques des gènes additionnels sont données dans le tableau 1 ci-dessous :

**[Table 1]**

| Gène (Nom) | Localisation chromosomique (GRCh38/hg38) | Numéro d'accession |
|---|---|---|
| | | NM_004355 |
| CD74 (Molécule CD74) | Chromosome 5: 150,400,041-150,412,969 | NM_001025158 |
| | | NM_001025159 |
| IL1R2 (Récepteur 2 de l'interleukine 1) | Chromosome 2: 101,991,960-102,028,544 | NM_001261419 |
| | | NM_004633 |
| CD177 (molécule CD177) | Chromosome 19: 43,353,686-43,363,172 | NM_020406 |

Selon une variante de ce mode de réalisation, le procédé comprend une étape de mesure, dans l'échantillon biologique du sujet, de l'expression du gène additionnel CD74. Selon cette variante, il peut être conclu à un risque de décès chez ledit sujet lorsque la comparaison du niveau de transcrits ARNm du gène TDRD9 avec une valeur seuil prédéterminée montre qu'il y a une augmentation du niveau d'expression ou une surexpression, et lorsque la comparaison du niveau de transcrits ARNm du gène additionnel CD74 avec une valeur seuil prédéterminée, montre qu'il y a une diminution du niveau d'expression ou une sous expression.

Selon une autre variante de ce mode de réalisation, le procédé comprend une étape de mesure, dans l'échantillon biologique du sujet, de l'expression du gène additionnel IL1R2. Selon cette variante, il peut être conclu à un risque de décès chez ledit sujet lorsque la comparaison du niveau de transcrits ARNm du gène TDRD9 avec une valeur seuil prédéterminée montre qu'il y a une augmentation du niveau d'expression ou une surexpression, et lorsque la comparaison du niveau de transcrits ARNm du gène additionnel IL1R2 avec une valeur seuil prédéterminée, montre qu'il y a une augmentation du niveau d'expression ou une surexpression.

Selon une autre variante de ce mode de réalisation, le procédé comprend une étape de mesure, dans l'échantillon biologique du sujet, de l'expression du gène additionnel CD177. Selon cette variante, il peut être conclu à un risque de décès chez ledit sujet lorsque la comparaison du niveau de transcrits ARNm du gène TDRD9 avec une valeur seuil prédéterminée montre qu'il y a une augmentation du niveau d'expression ou une surexpression, et lorsque la comparaison du niveau de transcrits ARNm du gène additionnel CD177 avec une valeur seuil prédéterminée, montre qu'il y a une augmentation du niveau d'expression ou une surexpression.

Selon une variante préférée de ce mode de réalisation, le procédé comprend une étape de mesure, dans l'échantillon biologique du sujet, de l'expression des gènes additionnels CD74 et IL1R2. Selon cette variante, il peut être conclu à un risque de décès chez ledit sujet lorsque la comparaison du niveau de transcrits ARNm du gène TDRD9 avec une valeur seuil prédéterminée montre qu'il y a une augmentation du niveau d'expression ou une surexpression, lorsque la comparaison du niveau de transcrits ARNm du gène additionnel CD74 avec une valeur seuil prédéterminée montre qu'il y a une diminution du niveau d'expression ou une sous expression, et lorsque la comparaison du niveau de transcrits ARNm du gène additionnel IL1R2 avec une valeur seuil prédéterminée montre qu'il y a une augmentation du niveau d'expression ou une surexpression. Selon cette variante préférée, les expressions augmentées de TDRD9 et IL1R2 et l'expression diminuée de CD74 sont significativement associées au décès du sujet dans les 28 jours suivants l'infection par le virus respiratoire. Toujours selon cette variante, les chances de survie du sujet sont d'environ 90% lorsque les expressions de TDRD9 et IL1R2 sont diminuées et l'expression de CD74 augmentée.

Selon une autre variante de ce mode de réalisation, le procédé comprend une étape de mesure, dans l'échantillon biologique du sujet, de l'expression des gènes additionnels CD74 et CD177. Selon cette variante, il peut être conclu à un risque de décès chez ledit sujet lorsque la comparaison du niveau de transcrits ARNm du gène TDRD9 avec une valeur seuil prédéterminée montre qu'il y a une augmentation du niveau d'expression ou une surexpression, lorsque la comparaison du niveau de transcrits ARNm du gène additionnel CD74 avec une valeur seuil prédéterminée montre qu'il y a une diminution du niveau d'expression ou une sous expression, et lorsque la comparaison du niveau de transcrits ARNm du gène additionnel CD177 avec une valeur seuil prédéterminée montre qu'il y a une augmentation du niveau d'expression ou une surexpression.

Selon une autre variante de ce mode de réalisation, le procédé comprend une étape de mesure, dans l'échantillon biologique du sujet, de l'expression des gènes additionnels CD177 et IL1R2. Selon cette variante, il peut être conclu à un risque de décès chez ledit sujet lorsque la comparaison du niveau de transcrits ARNm du gène TDRD9 avec une valeur seuil prédéterminée montre qu'il y a une augmentation du niveau d'expression ou une surexpression, et lorsque la comparaison du niveau des transcrits ARNm des gènes additionnels CD177 et IL1R2 avec des valeurs seuils prédéterminées, montre qu'il y a une augmentation des niveaux d'expression ou une surexpression desdits gènes additionnels.

Selon une autre variante de ce mode de réalisation, le procédé comprend une étape de mesure, dans l'échantillon biologique du sujet, de l'expression des gènes additionnels CD74, CD177 et IL1R2. Selon cette variante, il peut être conclu à un risque de décès accru chez ledit sujet lorsque la comparaison du niveau de transcrits ARNm du gène TDRD9 avec une valeur seuil prédéterminée montre qu'il y a une augmentation du niveau d'expression ou une surexpression, lorsque la comparaison du niveau de transcrits ARNm du gène additionnel CD74 avec une valeur seuil prédéterminée montre qu'il y a une diminution du niveau d'expression ou une sous expression, et lorsque la comparaison du niveau des transcrits ARNm des gènes additionnels CD177 et IL1R2 avec des valeurs seuils prédéterminées, montre qu'il y a une augmentation des niveaux d'expression ou une surexpression desdits gènes additionnels CD177 et IL1R2.

Selon ce mode de réalisation particulier, le procédé peut ainsi comprendre les étapes suivantes de :
- déterminer le niveau d'expression des gènes TDRD9 et CD74 par mesure de la quantité d'au moins un transcrit de TDRD9 et de CD74 dans l'échantillon biologique du sujet,
- comparer la quantité desdits transcrits déterminée pour ledit échantillon biologique ou une valeur dérivée de cette quantité, à une valeur de référence prédéterminée à partir d'un ou plusieurs échantillons de référence, et
- établir une conclusion quant au risque de décès, à partir du résultat de la comparaison.

Selon ce mode de réalisation, le procédé permet de conclure à un risque de décès du sujet lorsqu'une surexpression de TDRD9 et une sous expression de CD74 est mise en évidence dans l'échantillon biologique à tester.

Selon un mode de réalisation particulier, le risque de décès correspond au risque de décès du sujet dans les 2, 3, 4, 5, 6, 7, 8, 9, 10, 11, 12, 13, 15, 15, 16, 17, 18, 19, 20, 21, 22, 23, 24, 25, 26, 27 ou 28 jours post infection du sujet. De préférence, le risque de décès correspond au risque de décès du sujet dans les 7 jours, 14, jours, 21 jours, 22 jours, 23 jours, 24 jours, 25 jours, 26 jours, 27 jours, ou les 28 jours post infection du sujet. De préférence encore, le risque de décès correspond au risque de décès dans les 28 jours post-infection. On entend par le nombre de jours post infection, le nombre de jours suivant l'identification du sujet comme étant positif à une infection par un virus respiratoire.

Le procédé tel que décrit précédemment, dans tous ses modes de réalisation, peut également comprendre, outre l'étape de mesure de l'expression du(des) gène(s) décrits précédemment, une étape de mesure dans l'échantillon biologique du sujet de l'expression d'un ou plusieurs autres gènes additionnels , lesdits gènes étant impliqués dans la réponse de l'hôte et choisis parmi le panel de gènes présentés dans le tableau 2 ci-dessous.

**[Table 2]**

| Gène | Nom | Numéro d'accession |
|---|---|---|
| ADGRE3 | Adhésion G protein-coupled receptor E3 | NM_032571 |
| ARL14EP | Ribosylation factor like GTPase 14 effector protein | NM_152316 |
| BPGM | Biphosphoglycerate mutase | NM_199186 |
| | | NM_001293085 |
| | | NM_001724 |
| C3AR1 | Complement C3a receptor 1 | NM_004054 |
| CCNB1IP1 | Cyclin B1 interacting protein 1 | NM_182852 |
| CD274 | CD274 molecule | NM_014143 |
| | | NM_001267706 |
| CD3D | CD3d molecule | NM_000732 |
| | | NM_001040651 |
| CIITA | Class II major histocompatibility complex transactivator | NM000246 |
| | | NM_001286402 |
| | | NM_001286403 |
| CTLA4 | Cytotoxic T-lymphocyte associated protein 4 | NM_005214 |
| | | NM_001037631 |
| CX3CR1 | C-X3-C motif chemokine receptor 1 | NM_001337 |
| | | NM_001171171 |
| | | NM_001171172 |
| | | NM_001171174 |
| GNLY | Granulysin | NM_012483 |
| IFNG | Interferon gamma | NM_000619 |
| IL10 | Interleukin 10 | NM_000572 |
| IL1RN | Interleukin 1 receptor antagonist | NM_173842 |
| IL7R | Interleukin 7 receptor | NM_002185 |
| IP10/ CXCL10 | Interferon gamma induced protein 10 | NM_001565 |
| MDC1 | Mediator of DNA damage checkpoint 1 | NM_014641 |
| OAS2 | 2'-5'-oligoadenylate synthetase 2 | NM_001032731 |
| | | NM_016817 |
| | | NM_002535 |
| S100A9 | S100 calcium binding protein A9 | NM_002965 |
| TAP2 | Transporter 2, ATP binding cassette subfamily B member | NM_018833 |
| | | NM_001290043 |
| | | NM_000544 |
| TNF | Tumor necrosis factor | NM_000594 |
| ZAP70 | Zeta chain of T cell receptor associated protein kinase 70 | NM207519 |

Un autre objet de l'invention concerne un kit pour la mesure *in vitro* ou ex *vivo* du niveau d'expression du gène TDRD9 dans un échantillon biologique d'un sujet, ledit kit comprenant au moins un moyen de détermination du niveau d'expression de TDRD9 dans ledit échantillon biologique.

Les moyens de détermination du niveau d'expression du gène TDRD9 sont connus de l'homme du métier et peuvent être des outils ou réactifs spécifiques permettant de mesurer ladite expression dans un échantillon biologique. Il peut par exemple s'agir d'amorces ou des sondes.

Les modes de réalisation spécifiques ou préférés décrits en lien avec les procédés selon l'invention s'appliquent, bien entendu, aux kits et utilisations faisant l'objet de l'invention.

On entend par « amorce » ou « amorce d'amplification », un fragment nucléotidique pouvant être constitué de 5 à 100 nucléotides, de préférence de 15 à 30 nucléotides, et possédant une spécificité d'hybridation avec une séquence nucléotidique cible, dans des conditions déterminées pour l'initiation d'une polymérisation enzymatique, par exemple dans une réaction d'amplification enzymatique de la séquence nucléotidique cible. Généralement, on utilise des « couples d'amorces », constitués de deux amorces. Lorsque l'on souhaite réaliser l'amplification de plusieurs biomarqueurs (e.g des gènes) différents, plusieurs couples d'amorces différents sont de préférence utilisés, ayant préférentiellement chacun une capacité à s'hybrider spécifiquement avec un biomarqueur différent.

L'homme du métier est ainsi en mesure à partir de la séquence du gène et notamment des transcrits correspondants, de déterminer les amorces et les sondes nécessaires pour l'amplification, notamment pour l'amplification d'un ou plusieurs transcrits ARNm dudit gène.

On entend par « sonde » ou « sonde d'hybridation », un fragment nucléotidique constitué typiquement de 5 à 100 nucléotides, de préférence de 15 à 90 nucléotides, de manière encore plus préférée de 15 à 35 nucléotides, possédant une spécificité d'hybridation dans des conditions déterminées pour former un complexe d'hybridation avec une séquence nucléotidique cible. La sonde comporte également un rapporteur (tel qu'un fluorophore, une enzyme ou tout autre système de détection), qui va permettre la détection de la séquence nucléotidique cible. Dans la présente invention, la séquence nucléotidique cible peut être une séquence nucléotidique comprise dans un ARN messager (ARNm) ou une séquence nucléotidique comprise dans un ADN complémentaire (ADNc) obtenu par transcription inverse dudit ARNm. Lorsque l'on souhaite cibler plusieurs biomarqueurs (e.g. des gènes) différents, plusieurs sondes différentes sont de préférence utilisées, ayant préférentiellement chacune une capacité à s'hybrider spécifiquement avec un biomarqueur différent.

Par « hybridation », on entend le processus au cours duquel, dans des conditions appropriées, deux fragments nucléotidiques, tels que par exemple une sonde d'hybridation et un fragment nucléotidique cible, ayant des séquences suffisamment complémentaires, sont susceptibles de former un double brin avec des liaisons hydrogènes stables et spécifiques. Un fragment nucléotidique "capable de s'hybrider" avec un polynucléotide est un fragment pouvant s'hybrider avec ledit polynucléotide dans des conditions d'hybridation, qui peuvent être déterminées dans chaque cas de façon connue. Les conditions d'hybridation sont déterminées par la stringence, c'est-à-dire la rigueur des conditions opératoires. L'hybridation est d'autant plus spécifique qu'elle est effectuée à plus forte stringence. La stringence est définie notamment en fonction de la composition en bases d'un duplex sonde/cible, ainsi que par le degré de mésappariement entre deux acides nucléiques. La stringence peut également être fonction des paramètres de la réaction, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépendra principalement des sondes d'hybridation utilisées. Toutes ces données sont bien connues et les conditions appropriées peuvent être déterminées par l'homme du métier.

En général, selon la longueur des sondes d'hybridation utilisées, la température pour la réaction d'hybridation est comprise entre environ 20 et 70°C, en particulier entre 35 et 65°C dans une solution saline à une concentration d'environ 0,5 à 1 M. On réalise ensuite une étape de détection de la réaction d'hybridation.

Selon un mode de réalisation particulier, le kit comprend, en outre, au moins une donnée valeur seuil stockée sur un support lisible par un ordinateur, par exemple un code barre, et/ou utilisée sous la forme d'un code exécutable par un ordinateur configuré pour comparer le niveau de transcrits de TDRD9 déterminé grâce au moyen de détermination, ou une donnée obtenue à partir dudit niveau de transcrits du gène TDRD9, à ladite valeur seuil.

Selon ce mode de réalisation, la valeur seuil peut correspondre au niveau de transcrits, de préférence au niveau d'ARNm, de TDRD9 chez un sujet ne présentant aucune infection par un virus respiratoire ou présentant une infection par un virus respiratoire mais ayant survécu dans les 28 jours suivants l'infection. La valeur seuil peut également correspondre à la moyenne du niveau de transcrits, de préférence au niveau d'ARNm, de TDRD9 chez une population sujets ne présentant aucune infection par un virus respiratoire ou présentant une infection par un virus respiratoire mais ayant survécu dans les 28 jours suivants l'infection.

Selon un autre mode de réalisation, le kit comprend, en outre, au moins un niveau de référence pour ledit gène marqueur, stocké sur un support lisible par un ordinateur, par exemple un code barre, et/ou utilisé sous la forme d'un code exécutable par un ordinateur configuré pour comparer le niveau de transcrits de TDRD9 déterminé grâce au moyen de détermination audit niveau de référence, avec ledit niveau de référence qui est, de préférence, le niveau de transcrits de TDRD9, chez un sujet non infecté par un virus respiratoire, ou chez une population de tels sujets.

Selon un autre mode de réalisation, le kit comprend en outre un échantillon contrôle positif qui est un échantillon calibré pour contenir la quantité de TDRD9 qui correspond à la quantité ou concentration représentative du niveau d'expression mesuré dans un pool d'échantillons de sujets dont on sait qu'ils ne présentent pas d'infection par un virus respiratoire, et/ou un échantillon contrôle négatif qui est un échantillon calibré pour contenir la quantité de TDRD9 qui correspond à la quantité moyenne mesurée dans un pool d'échantillons de sujets dont on sait qu'ils n'ont pas survécu, notamment dans les 28 jours, suite à une infection par un virus respiratoire.

Selon un autre mode de réalisation, le kit comprend en outre un échantillon contrôle positif qui est un échantillon calibré pour contenir la quantité de TDRD9 qui correspond à la quantité ou concentration représentative du niveau d'expression mesuré dans un pool d'échantillons de sujets infectés par un virus respiratoire et dont on sait qu'ils ont survécu après 28 jours suivant l'infection, et/ou un échantillon contrôle négatif qui est un échantillon calibré pour contenir la quantité de TDRD9 qui correspond à la quantité moyenne mesurée dans un pool d'échantillons de sujets dont on sait qu'ils n'ont pas survécu, notamment dans les 28 jours, suite à une infection par un virus respiratoire.

Le kit, dans tous ses modes de réalisation, peut également comprendre au moins un moyen additionnel de détermination du niveau d'expression de gènes additionnels CD74, IL1R2 et/ou CD177, dans ledit échantillon biologique. Selon ce mode de réalisation, le kit peut comprendre, de la même manière que définie précédemment, une valeur seuil du niveau d'expression des gènes additionnels et/ou des échantillons contrôles (positifs ou négatifs) desdits gènes additionnels. Les moyens de détermination du niveau d'expression peuvent être des outils ou réactifs spécifiques tels que définis précédemment, par exemple des amorces ou des sondes.

Selon ce mode de réalisation, le kit peut comprendre, de la même manière que définie précédemment, une valeur seuil du niveau d'expression des gènes additionnels et/ou des échantillons contrôles (positifs ou négatifs) desdits gènes additionnels. Les moyens de détermination du niveau d'expression peuvent être des outils ou réactifs spécifiques tels que définis précédemment, par exemple des amorces ou des sondes.

Selon un mode de réalisation particulier, le kit, dans tous ses modes de réalisation, peut également comprendre, outre un moyen de détermination du niveau d'expression de TDRD9 et éventuellement des gènes additionnels CD74, IL1R2 et/ou CD177, au moins un autre moyen additionnel de détermination du niveau d'expression d'un ou plusieurs gènes impliqués dans la réponse de l'hôte, à partir d'un échantillon biologique d'un sujet. Les gènes impliqués dans la réponse de l'hôte sont tels que définis dans le panel de gènes présentés dans le tableau 2.

Les moyens de détermination du niveau d'expression peuvent être des outils ou réactifs spécifiques tels que définis précédemment, par exemple des amorces ou des sondes.

Un autre objet concerne l'utilisation d'un kit de mesure *in vitro* ou *in vivo* tel que défini précédemment pour déterminer le risque de décès chez un sujet infecté par un virus respiratoire.

Selon un mode de réalisation particulier, le kit selon l'invention, par la mesure additionnelle du niveau d'expression d'un panel de gènes impliqués dans la réponse de l'hôte, peut avantageusement être utilisé, outre la détermination du risque de décès suite à une infection par un virus respiratoire, pour la détermination du statut immunitaire d'un sujet afin de définir notamment si ledit sujet est immunocompétent ou immunodéprimé.

L'invention concerne également les procédés de détermination in vitro ou in vivo pour déterminer le risque de décès chez un sujet infecté par un virus respiratoire tels que définis par la présente description, qui comprennent en outre une étape de traitement de l'infection dudit virus respiratoire.

En particulier, le traitement peut être initié, dès lors qu'il est conclu au risque de décès chez ledit sujet infecté par un virus respiratoire.

Le traitement peut consister à l'administration d'un médicament antiviral adapté. A titre de traitement antiviral, on peut notamment citer l'Iopinavir^{®}, le Ritonavir^{®}, les interferons recombinants, notamment l'interferon beta, alpha et lambda. De nombreux traitements thérapeutiques de virus respiratoires, notamment ceux responsables de la COVID-19, sont actuellement en cours de test (Canedo-Marroquín, G. et al., 2020).

Selon un mode de réalisation particulier, l'étape de traitement consiste en l'administration d'un ou plusieurs anticorps monoclonaux audit sujet. Des exemples de tels anticorps sont le casirivimab, l'imdevimab, le regdanvimab, le tixagevimab ou le cilgavimab. Des traitements préférés peuvent consister en la combinaison du casirivimab et du l'imdevimab (Ronapreve), ou en la combinaison du tixagevimab et du cilgavimab.

Un autre objet concerne un procédé comprenant les étapes suivantes:
- obtenir un échantillon biologique de sang, de préférence de sang total, d'un sujet infecté par un virus respiratoire qui est le SARS-CoV-2,
- mettre en contact ledit échantillon biologique avec des réactifs spécifiques des produits d'expression des gènes cibles TDRD9, IL1R2, et CD177 et optionnellement, également l'expression du gène cible additionnel CD74
- mesurer l'expression desdits gènes cibles.

Les réactifs spécifiques des produits d'expression sont sélectionnés parmi des amorces d'amplification, des sondes d'hybridation ou des anticorps, et sont tels que définis précédemment.

Selon un mode de réalisation particulier, le procédé peut comprendre une étape d'administration d'un médicament antiviral adapté tel que défini précédemment, notamment lorsque l'expression des gènes cibles indique que le sujet présente un risque de décès dans les 28 jours.

Un autre objet concerne un procédé pour déterminer si un patient infecté par un virus respiratoire présente un risque de décès accru comprenant les étapes suivantes de :
- obtenir un échantillon biologique, de préférence de sang total, d'un patient infecté par un virus respiratoire,
- mesurer le niveau d'expression du gène TDRD9 dans ledit échantillon biologique,
- comparer le niveau d'expression du gène TDRD9 avec une valeur de référence obtenue à partir de patients infectés par un virus respiratoire et qui ont survécu,
   dans lequel, si le niveau d'expression du gène TDRD9 est supérieur à la valeur de référence, il est déterminé que le patient présente un risque accru de décès.

Selon un mode de réalisation particulier, le procédé comprend également la mesure du niveau d'expression d'un ou plusieurs gènes additionnels tels que définis précédemment et la comparaison des niveaux mesurés avec des valeurs de références respectives de chaque gènes additionnels également obtenues à partir de patients infectés par un virus respiratoire et qui ont survécu. Dans ce cas, la détermination du risque de décès accru est faite sur la base du résultat de la comparaison des niveaux d'expression de l'ensemble des gènes mesurés et telle que définie précédemment.

Enfin, un autre objet concerne un procédé comprenant la mesure quantitative, notamment par RT-qPCR, des ARNm des gènes TDRD9, IL1R2 et CD177 dans un échantillon biologique de sang d'un sujet infecté par un virus respiratoire tel que le SARS-CoV-2.

Selon un mode de réalisation particulier, le procédé comprend en outre la mesure quantitative de l'ARNm du gène CD74, notamment par RT-qPCR.

La présente invention est illustrée de manière non limitative à partir des exemples suivants.

### Exemples

### Matériel et Méthode

### Description des deux cohortes de patients utilisées

La cohorte RICO est une étude clinique d'observation prospective. A partir de cette cohorte, une sous-cohorte de 53 patients (ci-après sous-cohorte RICO) a été constituée par un recrutement entre mars et mai 2020 au sein de trois unités de soins intensifs d'hôpitaux universitaires (Hospices Civils de Lyon, Lyon, France). Les patients présentaient tous une infection pulmonaire à SARS-CoV-2 confirmée par un test RT-PCR.

Brièvement, les critères d'inclusion étaient les suivants (1) homme ou femme ≥ 18 ans, (2) hospitalisation en unités de soins intensifs (USI) pour une pneumopathie à SARS-CoV-2, (3) première hospitalisation en USI, (4) diagnostic positif d'infection à SARS-CoV-2 réalisé par PCR ou par une autre méthode approuvée dans au moins un échantillon respiratoire, (5) prélèvement dans les premières 24h après l'admission en USI (J0) réalisable et (6) patient ou proche parent ayant été informé des termes de l'étude et ne s'étant pas opposé à sa participation.

Les critères d'exclusion étaient la grossesse, les patients institutionnalisés et l'incapacité à obtenir le consentement éclairé.

A partir de cette sous-cohorte, les échantillons ont été prélevés au jour 0 (dans les 48 heures suivant l'admission en soins intensifs), ce qui représente 50 patients. Les patients étaient âgés en moyenne de 64 ans [InterQuartile Range, 52-70] et présentaient une distribution disparate d'hommes et de femmes (80/20).

Cohorte REALISM: Un sous-ensemble de 34 échantillons d'ARN de volontaires sains a été obtenu rétrospectivement à partir de la cohorte REALISM qui est une étude observationnelle longitudinale et monocentrique. Les échantillons de l'étude REALISM ont été collectés entre décembre 2015 et juin 2018. Cette sous-cohorte a été utilisée comme un ensemble de données de contrôle. Les volontaires sains étaient âgés de 63,5 ans [IQR, 52,0-70,0] et présentaient une distribution standard d'hommes et de femmes (50/50).

### Mesures des marqueurs immunologiques

Les marqueurs immunologiques suivants sont communs aux deux sous-cohortes : Nombre de lymphocytes T, nombre de lymphocytes T CD4+, nombre de lymphocytes T CD8+, mHLA-DR (Ac/C), plasma IL-6 et plasma IL-10. D'autres mesures de marqueurs immunologiques ont été effectuées uniquement dans la sous-cohorte RICO.

Les concentrations plasmatiques d'IL-6, TNF-a, IFN-γ et d'IL-10 ont été mesurées par la technologie Simpleplex^{®} en utilisant l'instrument ELLA selon les instructions du fabricant. Les concentrations plasmatiques d'IFNa2 ont été déterminées par single-molecule array (SIMOA) sur un analyseur HD-1 (Quanterix) en utilisant un kit commercial pour la quantification de l'IFN-α2. La teneur en IFN a été obtenue à l'aide de la technologie d'analyse nCounter^{®} (NanoString Technologies, Seattle) en calculant la médiane du compte normalisé des 6 gènes stimulés par l'interféron (ISG) suivants : SIGLEC1, IFI27, IFI44L, IFIT1, ISG15 et RSAD2. L'immunophénotypage de la sous-population des lymphocytes T a été réalisé sur un cytomètre en flux volumétrique automatisé de Beckman Coulter (Aquios CL). L'expression de HLA-DR par les monocytes a été réalisée à l'aide d'anticorps provenant de Beckman-Coulter et BD Biosciences (San Jose, USA). L'expression de HLA-DR des monocytes a été déterminée à l'aide du test Anti-HLA-DR/Anti-Monocyte Quantibrite (BD Biosciences, San Jose, USA). Le nombre total d'anticorps liés par cellule, exprimé en Ac/C, a été quantifié en utilisant la calibration avec une courbe standard déterminée avec les billes BD Quantibrite phycoerythrin (PE)(BD Biosciences).

Des échantillons de sang ont été collectés dans des tubes PAXgene^{®} (ref. 762165, PreAnalytiX GmbH Hombrechtikon Switzerland ) à J0 (48h post admission UCI).

L'ARN total a été extrait des tubes PAXgene^{™} à l'aide du kit Maxwell^{®} 16 LEV simplyRNA Blood (Promega), conformément aux directives du fabricant. La quantité d'ARN a été déterminée à l'aide d'un NanoVue (Biochrom).

Ensuite, 200 ng d'ARN total ont été testés en utilisant une poche FilmArray^{®} optimisée pour détecter un panel de gènes impliqués dans la réponse de l'hôte, dont TDRD9, par nested PCR. Les poches ont été analysées sur l'instrument FilmArray^{®} Torch (BioFire^{®}, USA) qui réalise de manière automatique les étapes d'extraction des acides nucléiques, de reverse-transcription et de qPCR.

Les valeurs d'expression normalisées des marqueurs (par rapport aux gènes de références DECR1, HPRT1 et PPIB) ont été calculées et utilisées pour les analyses.

### Analyse statistique

Les données qualitatives ont été rapportées sous forme de nombres ou de concentrations et les données quantitatives ont été rapportées sous forme de médiane [intervalle IQR]. Les caractéristiques cliniques ou immunologiques des volontaires de la sous-cohorte RICO et les volontaires sains (cohorte REALISM) ont été comparées à l'aide du test non paramétrique de Mann-Whitney-Wilcoxon pour les variables continues ou du test exact de Fisher ou du test du chi carré pour les variables catégorielles. Le niveau de signification a été fixé à 5 % pour les tests bilatéraux. Les analyses statistiques ont été réalisées à l'aide du logiciel R, version 4.0.4. Les données ont été centrées pour réaliser une analyse en composantes principales non supervisée.

La corrélation entre les marqueurs d'expression génique et les paramètres immunologiques a été évaluée à l'aide des scores de corrélation p de Spearman. Tout le panel de gène de la poche FilmArray^{®} a été utilisé, sans appliquer de sélection de caractéristiques, afin de construire cinq modèles de prédiction de la survie à 28 jours, à savoir : Elastic net, LASSO, Ridge régression, random forest (rf) et Partial Least Square Discriminant Analysis (PLS) en utilisant le package CARET (version 6.0-88). Les hyperparamètres ont été réglés et les modèles ont été ajustés en utilisant la validation croisée (k-fold=3, nombre de répétitions=10) dans la sous-cohorte RICO.

En résumé, parmi les 5 algorithmes d'apprentissage automatique évalués, Elastic net (α=0,1, λ=0,89898) était le plus performant avec une aire sous la courbe ROC (AUC) de 0,675 [intervalle de confiance], le LASSO (α=1, λ=0. 112) s'est classé deuxième avec une AUC de 0,671, puis le PLS (ncomp=1) avec une AUC de 0,668, le modèle Ridge régression (α=0, λ=1) était quatrième avec une AUC de 0,650 et enfin, le Random Forest (mtry=5) est arrivé dernièr en termes de performance avec une AUC de 0,63.

Les biomarqueurs d'intérêts ont été identifiés par l'importance des variables portées par chaque modèle dans la survenue de l'événement mortalité à 28 jours. Ces biomarqueurs identifiés par les algorithmes d'apprentissage automatiques sont les mêmes que ceux qui résultent significativement de l'analyse univariée d'association à la mortalité à 28 jours. Les patients ont été stratifiés en 2 groupes sur la base du seuil de Youden, déterminé pour chaque biomarqueur, pour la prédiction de la mortalité à 28 jours dans la population totale des patients à l'admission et les événements cumulatifs non ajustés de décès ont été obtenus avec les estimations de Kaplan-Meier.

### Ethique

Le protocole de l'étude REALISM a été approuvé par l'IRB (Comité de Protection des Personnes Sud-Est Il numéro d'approbation 2015-42-2). Cette étude clinique a également été enregistrée sur clinicaltrials.gov (NCT02638779). Des consentements éclairés ont été obtenus pour chaque patient et/ou auprès des proches parents, les patients ou les représentants légaux ont été informés de l'étude et de leur droit de refuser de participer.

Le protocole de l'étude RICO a été approuvé par le comité d'éthique (Comité de Protection des Personnes Ile de France 1 - N°IRB/IORG # : IORG0009918) sous le numéro d'accord 2020-A01079-30. Cette étude clinique a été enregistrée sur ClinicalTrials.gov (NCT04392401). Le comité a renoncé à la nécessité d'un consentement éclairé écrit car il s'agissait d'une étude d'observation, présentant un faible risque pour les patients, et aucune procédure spécifique, autre qu'un prélèvement sanguin de routine, n'était requise.

Les deux cohortes sont conformes à la Déclaration d'Helsinki, aux principes des bonnes pratiques cliniques et à la loi française sur la protection des données personnelles.

### Résultats

### Caractéristiques cliniques à l'admission en unité de soins intensifs

Les caractéristiques des patients de la sous-cohorte RICO et des volontaires sains recrutés sont présentées dans le **tableau 3** ci-dessous. Les patients ont été hospitalisés dans 3 hôpitaux de Lyon pendant la période qui a coïncidé avec la première vague de cas de COVID-19 en France (mars - mai 2020).

Brièvement et comme précédemment mentionné, 80% des patients étaient des hommes. Les patients ont été admis à l'hôpital avec une médiane de 8 jours après la présentation des premiers symptômes [IQR, 5-10], sans différence significative entre les survivants et les non-survivants à 28 jours. 44% des patients présentaient au moins une comorbidité, le diabète étant la plus fréquente (13/22 patients). Les patients présentaient un indice de masse corporelle (IMC) médian (kg/m3) de 29,33 [IQR, 26,58-31,54], sans différence entre les survivants et les non-survivants.

En termes de gravité de la maladie, les patients présentaient une PaO₂/FiO₂ élevée [médiane : 134 ; IQR : 90,5-191] et un score SAPS II [médiane : 33,5 ; IQR : 24,5-43,25]. Parmi les 50 patients, 11 (22 %) sont décédés au 28e jour.

À noter que les non-survivants présentaient un score SAPS II significativement plus élevé [médiane : 39 ; IQR : 34-53,5] par rapport aux survivants [médiane : 32 ; IQR : 22-39,5] (valeur p = 0,023).

Dans l'ensemble, les survivants ont passé une durée médiane de 30 jours [IQR, 13,25-62,5] à l'hôpital, dont 10 jours [IQR, 3-40] en soins intensifs. La moitié de cette sous-cohorte RICO a développé des infections secondaires (52%), sans différence entre les survivants et les non-survivants.

**[Table 3]**

| | | **Patients (n=50)** | **Survivant à 28 jours (n=39)** | **Non-survivant à 28 jours (n=11)** | ***p* value** |
|---|---|---|---|---|---|
| **Démographie** | | | | | |
| Age | | 64 [52-70] | 63 [51-69.5] | 67 [60-78] | 0.096 |
| Genre, Homme | | 40 (80%) | 30 (76.92%) | 10 (90.91%) | 0.424 |
| IMC [kg/m^{3]} | | 29.33 [26.58-31.54] | 29.41 [26.86-32.2] | 28.28 [24.45-29.91] | 0.292 |
| IMC > 30 kg/m³ | | 19 (38%) | 16 (41.03%) | 3 (27.27%) | 0.498 |
| **Comorbidités** | | | | | |
| Diabètes sucré | *Aucune* | 37 (74%) | 30 (76.92%) | 7 (63.64%) | |
| | *Avec détérioration* | 3 (6%) | 1 (2.56%) | 2 ( 18.18%) | 0.197 |
| | *Sans détérioration organique* | 10 (20%) | 8 (20.51%) | 2 (18.18%) | |
| Comorbidités | >=1 | 22 (44%) | 16 (41.03%) | 6 (54.55%) | |
| | 0 | 28 (56%) | 23 (58.97%) | 5 (45.45%) | 0.503 |
| Score Charlson | | 0 [0-2] | 0 [0-1] | 1 [0-2] | 0.243 |
| Symptômes à l'admission | | | | | |
| Délai entre les premiers symptômes [jours] | | 8 [5-10] | 8 [5.5-10.5] | 6 [4-7] | 0.079 |
| Score de sévérité | | | | | |
| SOFA Score | | 4 [2-8] | 3 [2-8] | 7 [4.5-8] | 0.068 |
| SAPS II Score | | 33.5 [24.5-43.25] | 32 [22-39.5] | 39 [34-53.5] | 0.023 |
| Ratio PaO₂/FIO₂ à l'admission | | 134^{a} [90.5-191] | 152.5^{b} [88.5-204.75] | 120 [101-135.5] | 0.429 |
| Thérapie antivirale | | | | | |
| Hydroxychloroquine | | 22 (62.86%) | 17 (60.71%) | 5 (71.43%) | 0.689 |
| Lopinavir/ritonavir | | 5 (14.29%) | 5 (17.86%) | 0 (0%) | 0.559 |
| Lopinavir/ritonavir + Interferon β | | 5 (14.29%) | 4 (14.29%) | 1 (14.29%) | 1 |
| Remdesivir | | 1 (2.86%) | 1 (3.57%) | 0 (0%) | 1 |
| Soutien aux organes | | | | | |
| Ventilation mécanique à l'admission | | 30 (60%) | 20 (51.28%) | 10 (90.91%) | 0.033 |
| Ventilation mécanique invasive | | 19 (38%) | 13 (33.33%) | 6 (54.55%) | 0.293 |
| Médicaments vaso-actifs | | 19 (38%) | 13 (33.33%) | 6 (54.55%) | 0.293 |
| Traitement de substitution rénale | | 10 (20%) | 7 (17.95%) | 3 (27.27%) | 0.671 |
| Suivi | | | | | |
| Jours en USI | | 9 [4-29.75] | 10 [3-40] | 8 [6-19] | 0.716 |
| Jours à l'Hôpital | | 21 [13-56] | 30 [13.25-62.5] | 15 [7.5-20] | 0.007 |
| Mortalité à 28 jours | | 11 (22%) | 0 (0%) | 11 (100%) | <0.001 |
| Mortalité à 90 jours | | 14 (28.57%) | 3 (7.89%) | 11 (100%) | <0.001 |
| Infections acquises en USI | | 26 (52%) | 19 (48.72%) | 7 (63.64%) | 0.501 |

Les médianes et les écarts interquartiles [Q1-Q3] sont indiqués pour les variables continues où les nombres et pourcentages sont présentés pour les variables catégorielles. Les patients de l'étude COVID-19 ont été séparés en deux groupes en fonction de leur statut de survie à 28 jours après l'admission. Les scores de défaillance organique séquentielle (SOFA) et de physiologie aiguë simplifiée II (SAPS II) ont été calculés au cours des 24 premières heures après l'admission. Les données ont été comparées à l'aide du test non paramétrique de Mann-Whitney pour les variables continues ou du test exact de Fisher pour les variables catégorielles. ^{a,b} Trois patients n'ont pas présenté de PaO₂/FiO₂, ce qui a donné lieu à 47 mesures (sur 50) dans la colonne de tous les patients et à 36 mesures (sur 39) chez les survivants à 28 jours.

### Association entre l'expression de biomarqueurs et le statut clinique des patients

La relation entre les marqueurs transcriptomiques de la poche FilmArray^{®} et les différents résultats cliniques a été analysée.

En premier lieu, l'association avec la gravité des patients à l'admission, telle que reflétée par les scores cliniques a été étudiée afin de confirmer le potentiel des biomarqueurs. Il ressort notamment que CD74 est négativement corrélé au score SAPSII (p = -0,30).

D'un autre côté, une expression augmentée de TDRD9, CD177, et IL1R2 était positivement corrélée avec le score SAPSII (TDRD9 : p = 0,46, CD177 : p = 0,5, et IL1R2 : p = 0,47).

### Association entre l'expression de biomarqueurs et la mortalité des patients à 28 jours

En utilisant 5 approches différentes d'apprentissage automatique, 4 gènes associés à la prédiction de la mortalité à 28 jours ont été identifiés. Il s'agit des gènes TDRD9, CD74, IL1R2 et CD177 **(****Figure 1****).**

Ces quatre marqueurs sont exprimés de manière différentielle dans une analyse univariée entre les patients survivants et les non-survivants (Tableau 4).

**[Table 4]**

| | **OR_{IQR} [95% Cl]** | **IQR** | **p value** |
|---|---|---|---|
| **TDRD9** | **4.221 [1.591-13.621]** | **2.01** | **0.007** |
| **CD177** | **4.085 [1.315-16.548]** | **3.58** | **0.027** |
| **CD74** | **0.202 [0.054-0.569]** | **1.14** | **0.007** |
| **IL1R2** | **3.548 [1.448-10.424]** | **2.28** | **0.010** |
| mHLA-DR [Ac/C] | 1.072 [0.435-2.402] | 7335 | 0.868 |
| Cellules T CD3 [cellules/µL] | 0.26 [0.054-0.816] | 362 | 0.050 |
| IL-6 Plasmatique [pg/µL] | 1.338 [1.034-2.3] | 142 | 0.187 |
| IL-10 Plasmatique [pg/µL] | 1.116 [0.945-1.407] | 16 | 0.221 |

L'association entre le statut de survie à 28 jours et les marqueurs transcriptomiques ou les paramètres immunitaires classiques a été réalisée en mettant en œuvre des modèles de régression logistique univariés et les odds ratio (OR) de l'intervalle interquartile (IQR) ont été extraits. Pour permettre la comparaison entre les modèles, les odds ratios (OR) calculés pour chaque paramètre immunitaire et transcriptomique ont été normalisés à un incrément du premier au troisième quartile. Les valeurs p ≤ 0,05 sont mises en évidence en gras.

Plus précisément, TDRD9 était significativement régulé à la hausse chez les patients non survivants par rapport aux patients survivants.

Concernant le niveau d'expression de gènes additionnels, IL1R2 et CD177 étaient significativement régulés à la hausse chez les patients non survivant par rapport aux patients survivants, tandis que CD74 était significativement régulé à la baisse chez les patients non survivants par rapport aux survivants et dans une plus large mesure par rapport aux volontaires sains.

En revanche, les paramètres immunologiques mesurés à l'admission (HLA-DR monocytaire, IL-6 et IL-10 plasmatiques) n'étaient pas significativement associés au statut de survie des patients à 28 jours **(****Figure 2****)** démontant ainsi tout le potentiel prédictif de TDRD9 et des marqueurs additionnels selon l'invention.

Sur la base de l'observation selon laquelle l'âge et la ventilation mécanique invasive sont associés à la gravité chez les patients atteints de la maladie COVID-19 (cdc.gov/coronavirus/2019-nCoV), ces paramètres ont été utilisés comme facteurs de confusion dans une analyse multivariée [3a]. Nous avons observé que le biomarqueur TDRD9 et les trois biomarqueurs additionnels CD74, IL1R2 et CD177 restaient significativement associés à la survie et présentaient un odds ratio (OR) interquartile de 5,35 pour TDRD9, 0,22 pour CD74, 3,26 pour IL1R2 et 5,22 pour CD177 (résultats non montrés).

Par l'intermédiaire des courbes de Kaplan-Meier avec l'expression de chaque gène utilisant le seuil de Youden (de prédiction de la survie des patients à 28 jours suivants l'infection) pour distinguer l'expression faible/élevé, il a été identifié que les expressions TDRD9^{élevé}, CD74^{faible}, CD177^{élevé} et IL1R2^{élevé} étaient significativement associés au décès sur une période de 28 jours, alors que les chances de survie étaient d'environ 90 % pour les patients présentant les expressions TDRD9^{faible}, CD74^{élevé}, CD177^{faible} et IL1R2^{faible} **(****Figure 3****).** Ces résultats démontrent ainsi de nouveau la valeur prédictive des biomarqueurs selon l'invention pour la détermination du risque de décès des patients infectés par un virus respiratoire de type SARS-Cov-2, notamment du risque de décès à 28 jours.

Les résultats obtenus ci-dessus avec le gène TDRD9, et les gènes additionnels ont été confirmés à partir de la cohorte RICO, plus précisément dans une seconde sous-cohorte de 306 patients recrutés entre août 2020 et août 2021 au sein des trois unités de soins intensifs d'hôpitaux universitaires (Hospices Civils de Lyon, Lyon, France).

### REFERENCES BIBLIOGRAPHIQUES

C. Huang, et al., "Clinical features of patients infected with 2019 novel coronavirus in Wuhan, China" Lancet, 2020, 395, 497, https://doi.org/10.1016/S0140-6736(20)30183-5;
E. Z. Ong, et al.," A Dynamic Immune Response Shapes COVID-19 Progression" Cell Host Microbe, 2020, 27 (6), 879, https://doi.org/10.1016/j.chom.2020.03.021;
F. Venet et al., « Longitudinal assessment of IFN-I activity and immune profile in critically ill COVID-19 patients with acute respiratory distress syndrome"; Crit Care, 2021, 25 (1), 140, https://doi.org/10.1186/s13054-021-03558-w;
D. Mathew, et al., « Deep immune profiling of COVID-19 patients reveals distinct immunotypes with therapeutic implications" ; Science, 2020, 369 (6508), https://doi.org/10.1126/science.abc8511 ;
A. G. Laing et al., "A dynamic COVID-19 immune signature includes associations with poor prognosis", Nat Med, 2020, 26 (10), 1623, https://doi.org/10.1038/s41591-020-1038-6.
M. S. Abers, et al., "An immune-based biomarker signature is associated with mortality in COVID-19 patients" JCI Insight, 2021, 6 (1):e144455, https://doi.org/10.1172/jci.insight.144455;
Guardela et al., "50-gene risk profiles in peripheral blood predict COVID-19 outcomes: A rétrospective, multicenter cohort study" EBioMedecine, 2021, https://doi.org/10.1016/j.ebiom.2021.103439;
Y. Levy et al., "CD177, a specific marker of neutrophil activation, is associated with coronavirus disease 2019 severity and death" Iscience, 2021, 24(7):102711 DOI: 10.1016/j.isci.2021.102711;
G. Canedo-Marroquín et al., "SARS-CoV-2: Immune Response Elicited by Infection and Development of Vaccines and Treatments" Front. Immunol, 2020, 11:569760; DOI 10.3389/fimmu.2020.569760.

## Revendications

1. Procédé *in vitro* ou ex *vivo* pour déterminer le risque de décès chez un sujet infecté par un virus respiratoire, comprenant une étape de mesure, dans un échantillon biologique dudit sujet, du niveau d'expression du gène TDRD9.

2. Procédé selon la revendication 1, **caractérisé en ce que** le virus respiratoire est le SARS-CoV-2 ou un de ses variants.

3. Procédé selon la revendication 1 ou 2, **caractérisé en ce qu'**il comprend en outre une étape de mesure dans l'échantillon biologique dudit sujet de l'expression d'au moins un autre gène additionnel choisi parmi CD74, IL1R2 et/ou CD177.

4. Procédé selon la revendication 1 ou 3, **caractérisé en ce que** l'échantillon biologique est un échantillon de sang, de préférence un échantillon de sang total.

5. Procédé selon l'une des revendications 1 à 4, **caractérisé en ce que** le l'expression du gène TDRD9 est mesurée au niveau ARN messager (ARNm).

6. Procédé selon l'une des revendications 1 à 5, **caractérisé en ce que** l'expression est mesurée par RT-PCR, de préférence par RT-qPCR, par séquençage, ou par hybridation.

7. Procédé selon la revendication 5 ou 6, **caractérisé en ce que** l'expression est normalisée par rapport à l'expression d'un ou plusieurs gènes de ménage.

8. Procédé selon l'une des revendications 1 à 7, **caractérisé en ce qu'**il comprend en outre les étapes suivantes de :
- comparer le niveau d'expression du gène TDRD9 pour ledit échantillon biologique ou une valeur dérivée de cette quantité, à une valeur de référence prédéterminée, et
- établir une conclusion quant au risque de décès, à partir du résultat de la comparaison.

9. Procédé selon la revendication 8, **caractérisé en ce que** la valeur de référence correspond à la moyenne du niveau d'expression de TDRD9 obtenu à partir d'échantillons biologiques issus d'une population de sujets ne présentant aucune infection par un virus respiratoire ou à la moyenne du niveau d'expression de TDRD9 obtenue à partir d'échantillons biologiques issus d'une population de sujets infectés par un virus respiratoire et dont on sait qu'ils ont survécus après l'infection, notamment dans les 28 jours suivants l'infection.

10. Procédé selon l'une des revendications 1 à 9, **caractérisé en ce qu'**il comprend également une étape de mesure dans l'échantillon biologique du sujet de l'expression d'un ou plusieurs autres gènes additionnels, lesdits gènes étant impliqués dans la réponse de l'hôte et choisis parmi le panel de gènes listés ci-après :
| Gène | Nom | Numéro d'accession |
|---|---|---|
| ADGRE3 | Adhésion G protein-coupled receptor E3 | NM_032571 |
| ARL14EP | Ribosylation factor like GTPase 14 effector protein | NM_152316 |
| BPGM | Biphosphoglycerate mutase | NM_199186 |
| | | NM_001293085 |
| | | NM_001724 |
| C3AR1 | Complement C3a receptor 1 | NM_004054 |
| CCNB1IP1 | Cyclin B1 interacting protein 1 | NM_182852 |
| CD274 | CD274 molecule | NM_014143 NM_001267706 |
| CD3D | CD3d molecule | NM_000732 |
| | | NM_001040651 |
| CIITA | Class II major histocompatibility complex transactivator | NM000246 |
| | | NM_001286402 |
| | | NM_001286403 |
| CTLA4 | Cytotoxic T-lymphocyte associated protein 4 | NM_005214 |
| | | NM_001037631 |
| CX3CR1 | C-X3-C motif chemokine receptor 1 | NM_001337 |
| | | NM_001171171 |
| | | NM_001171172 |
| | | NM_001171174 |
| GNLY | Granulysin | NM_012483 |
| IFNG | Interferon gamma | NM_000619 |
| IL10 | Interleukin 10 | NM_000572 |
| IL1RN | Interleukin 1 receptor antagonist | NM_173842 |
| IL7R | Interleukin 7 receptor | NM_002185 |
| IP10/ CXCL10 | Interferon gamma induced protein 10 | NM_001565 |
| MDC1 | Mediator of DNA damage checkpoint 1 | NM_014641 |
| OAS2 | 2'-5'-oligoadenylate synthetase 2 | NM_001032731 |
| | | NM_016817 |
| | | NM_002535 |
| S100A9 | S100 calcium binding protein A9 | NM_002965 |
| TAP2 | Transporter 2, ATP binding cassette subfamily B member | NM_018833 |
| | | NM_001290043 |
| | | NM_000544 |
| TNF | Tumor necrosis factor | NM_000594 |
| ZAP70 | Zeta chain of T cell receptor associated protein kinase 70 | NM207519 |

11. Kit pour la mesure in vitro ou ex vivo, de l'expression de TDRD9 dans un échantillon biologique comprenant des moyens de détermination du niveau d'expression de TDRD9 dans ledit échantillon, lesdits moyens étant des amorces ou des sondes.

12. Kit selon la revendication 11, **caractérisé en ce qu'**il comprend un échantillon contrôle positif calibré pour contenir la quantité de TDRD9 qui correspond à la quantité ou la concentration représentative du niveau d'expression mesuré dans un pool d'échantillons de sujets ne présentant pas d'infection par un virus respiratoire, et/ou un échantillon contrôle négatif calibré pour contenir la quantité de TDRD9 qui correspond à la quantité moyenne mesurée dans un pool d'échantillons de sujets qui n'ont pas survécu suite à une infection par un virus respiratoire.

13. Kit selon l'une des revendications 11 à 13, **caractérisé en ce qu'**il comprend des moyens de détermination du niveau d'expression d'au moins un autre gène additionnel sélectionné parmi CD74, IL1R2 et CD177.

14. Kit selon l'une des revendications 12 à 15, **caractérisé en ce qu'**il comprend des moyens de détermination du niveau d'expression d'un ou plusieurs gène additionnel impliqués dans la réponse de l'hôte tel que définis dans la revendication 10.

15. Utilisation du kit selon l'une des revendications 12 à 16 pour la détermination du risque de décès, de préférence du risque de décès dans les 28 jours post-infection, d'un sujet infecté par un virus respiratoire réplicatif tel que le SARS-CoV-2 ou un de ses variants.
